# EUROPEAN PATENT APPLICATION

(11) **EP 4 270 277 A1**
(43) Date of publication of application: **01.11.2023**
(21) Application number: 21910862.8
(22) Date of filing: 22.12.2021
(51) Int. Cl.: G06Q 10/06

(54) **WORK BEHAVIOR RECOGNITION SYSTEM AND WORK BEHAVIOR RECOGNITION METHOD**

(30) Priority: 25.12.2020 JP 2020217192
(71) Applicant: Hitachi, Ltd., Tokyo 100-8280 (JP)
(72) Inventor: AKIYAMA, Takayuki, Tokyo 100-8280 (JP)
(74) Representative: MERH-IP Matias Erny Reichl Hoffmann Patentanwälte PartG mbB
(86) International application number: PCT/JP2021/047583
(87) International publication number: WO 2022/138722

(57) **Abstract**

There are provided an action recognition section that recognizes an action of a worker on the basis of information detected by a sensor worn by the worker and a predetermined action recognition model; a work intensity determination section that determines work intensity of work performed by the worker, on the basis of the information detected by the sensor and a predetermined work intensity determination model; and a control section that associates the action of the worker obtained by the action recognition section with the work intensity of the work performed by the worker, the work intensity being obtained by the work intensity determination section, and outputs the association.

## Description

### Technical Field

The present invention relates to a work action recognition system and a work action recognition method.

### Background Art

Heretofore, there have been various techniques for measuring the load resulting from work postures. For example, Patent Document 1 describes techniques for estimating physical ardor in reference to a previously stored physical ardor-heart rate-acceleration database DB12c in which the physical ardor PA, heart rate HR, and three-axis acceleration (AccX, AccY, AccZ) are associated with each other with regard to each of multiple types of actions performed by test subjects. Patent Document 2 describes techniques for estimating the action status of a measurement target on the basis of a trained action estimation model and measured sensor data.

### Prior Art Document

### Patent Documents

Patent Document 1: JP-2017-143910-A
Patent Document 2: JP-2019-103609-A

### Summary of the Invention

### Problems to be Solved by the Invention

The measurement of the load resulting from posture fails to measure differences in work intensity in the same posture. The estimation based on heart rate and cardiac sensors entails differences in heath condition between individuals and between days. Furthermore, it is practically difficult to acquire beforehand the data regarding the types of work involving high levels of work intensity.

It is therefore an object of the present invention to provide a work action recognition system and a work action recognition method for estimating work intensity with no need to obtain beforehand the intensity of the work involved.

### Means for Solving the Problems

In solving the foregoing problems and achieving the foregoing object, , a work action recognition system according to the present invention includes: an action recognition section that recognizes an action of a worker on the basis of information detected by a sensor worn by the worker and a predetermined action recognition model; a work intensity determination section that determines work intensity of work performed by the worker, on the basis of the information detected by the sensor and a predetermined work intensity determination model; and a control section that associates the action of the worker obtained by the action recognition section with the work intensity of the work performed by the worker, the work intensity being obtained by the work intensity determination section, and outputs the association.

### Advantages of the Invention

The present invention makes it possible to estimate work intensity with no need to obtain beforehand the intensity of the work involved.

### Brief Description of the Drawings

FIG. 1 is a diagram depicting a typical configuration of a work action recognition system according to a first embodiment of the present invention.
FIG. 2 is a diagram depicting a typical processing procedure performed by the work action recognition system according to the first embodiment.
FIG. 3A is a tabular view indicating a typical model used by this system.
FIG. 3B is a tabular view indicating another typical model used by this system.
FIG. 3C is a tabular view indicating another typical model used by this system.
FIG. 4 is a diagram depicting a typical configuration of a work action recognition system according to a second embodiment of the present invention.
FIG. 5A is a diagram depicting a typical processing procedure performed by the work action recognition system according to the second embodiment.
FIG. 5B is a diagram depicting a typical screen (annotation panel) displayed on an information presentation device.
FIG. 5C is a tabular view indicating a typical work intensity generation model.
FIG. 5D is a diagram depicting a typical process of generating the work intensity generation model.
FIG. 6 is a tabular view indicating typical work action data.
FIG. 7 is a diagram depicting a typical configuration of a work action recognition system according to a third embodiment of the present invention.
FIG. 8 is a diagram depicting a typical processing procedure performed by the work action recognition system according to the third embodiment.
FIG. 9A is a tabular view indicating a typical work load score correspondence table.
FIG. 9B is a tabular view indicating another typical work load score correspondence table.
FIG. 10 is a diagram depicting another typical processing procedure performed by the work action recognition system according to the third embodiment.
FIG. 11 is a tabular view indicating a typical posture load score correspondence table.
FIG. 12 is a diagram depicting a typical screen (work load value screen) which indicates transitions of load (work load values) on the worker and which is output to the information presentation device.
FIG. 13 is a diagram depicting a typical configuration of a work action recognition system according to a fourth embodiment of the present invention.
FIG. 14A is a tabular view indicating a typical worker management database.
FIG. 14B is a tabular view indicating a typical work management database.
FIG. 15 is a diagram depicting a typical screen (load management screen) that outputs data stored in the worker management database indicated in FIG. 14A as well as data stored in the work management database in FIG. 14B.
FIG. 16 is a schematic diagram of a computer for use with each embodiment.

### Modes for Carrying Out the Invention

The preferred embodiments of the present invention are described below with reference to the accompanying drawings. The ensuing description and the drawings are examples intended to explain the present invention and are thus abbreviated and simplified as needed for clarification. This invention may also be embodied in various other forms. The components of the embodiment may each be formed by single or multiple elements unless otherwise specified.

The positions, sizes, shapes, and ranges of the components indicated in the drawings may not represent their actual positions, sizes, shapes, and ranges for the purpose of facilitating the understanding of the present invention. It is thus to be noted that the positions, sizes, shapes, and ranges disclosed in the drawings are not necessarily limitative of this invention.

In the description that follows, diverse information may be explained using expressions such as "table" and "list." However, such information may alternatively be expressed using other data structures. The expressions such as "XX table" or "XX list" may be referred to as "XX information" in order to indicate that the information is not dependent on data structures. In the case where identification information is explained using expressions such as "identification information," "identifier," "name," "ID," or "number," these expressions are interchangeable.

In the case where there are multiple components having the same or similar functions, these components may be explained using the same reference signs with different suffixes. However, if there is no need to distinguish these components, they may be explained without recourse to such suffixes.

In the description that follows, there are cases of explaining processes performed by executing programs. The programs are executed by a processor (e.g., CPU (Central Processing Unit) or GPU (Graphics Processing Unit)) in such a manner that predetermined processes are carried out using storage resources (e.g., memory) and/or an interface device (e.g., communication port) as needed. In that sense, the agent of the processing may be considered the processor. Likewise, the agent of the processing performed by program execution may be a controller, an apparatus, a system, a computer, or a node having the processor. The agent of the processing performed by executing the program may be an arithmetic section that may include a dedicated circuit for carrying out specific processes (e.g., FPGA (Field-Programmable Gate Array or ASIC (Application Specific Integrated Circuit)).

The programs may be installed into an apparatus such as a computer from a program source. The program source, for example, may be a program distribution server or computer-readable storage media. In the case where the program source is the program distribution server, the program distribution server may include a processor and storage resources for storing the programs targeted for distribution and the processor of the program distribution server may distribute the distribution target programs to other computers. In the ensuing description, two or more programs may be considered implemented as one program, and one program may be considered implemented as two or more programs.

### First embodiment

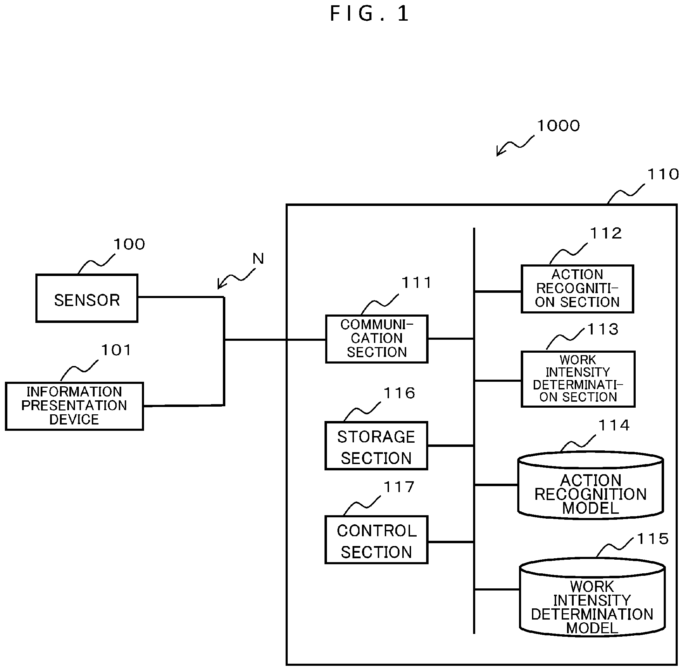

FIG. 1 is a diagram depicting a typical configuration of a work action recognition system 1000 according to a first embodiment of the present invention. As depicted in FIG. 1, the work action recognition system 1000 includes a sensor 100 that detects the work posture of a worker, an information presentation device 101 that outputs an estimated result of work intensity, and a computer 110 that performs various processes for estimating the work intensity of the worker using information detected by the sensor 100. These components are interconnected via a network N. The network N may be a common wired or wireless network such as the Internet.

The sensor 100 may be an acceleration sensor that can be worn by the worker, for example. The sensor 100 detects the work posture and work intensity of the worker wearing the sensor. Although the present embodiment adopts a work garment-type wearable sensor as the sensor 100 for example, some other wearable sensor may be used instead as long as it can detect the work posture and work intensity of the worker.

The information presentation device 101 may be an LCD (Liquid Crystal Display), for example, and displays the work intensity resulting from the estimation by the computer 110. The computer 110 is an information processing apparatus such as a smartphone or a PC (Personal Computer) that estimates the work intensity of the worker using the information detected by the sensor 100.

As depicted in FIG. 1, the computer 110 includes a communication section 111, an action recognition section 112, a work intensity determination section 113, an action recognition model 114, a work intensity determination model 115, a storage section 116, and a control section 117.

The communication section 111 may be a NIC (Network Interface Card), for example. The communication section 111 permits exchanges of diverse information between the computer 110 and other equipment.

The action recognition section 112 may be a program executed by the CPU, for example. The action recognition section 112 determines and recognizes the action of the worker on the basis of the action recognition model 114.

The work intensity determination section 113 may be a program executed by the CPU, for example. The work intensity determination section 113 determines, on the basis of the work intensity determination model 115, the intensity of the work performed by a recognized worker performing some kind of action. Here, work intensity is a concept indicative of a degree of action on the work target by the worker at a given point in time during work. For example, in the case where the worker handles a package as the work target, work intensity refers not only to the force required to carry the package but also to the effort of maintaining the posture of the worker holding the package. In another example where the worker handles the screw of a valve as the work target, work intensity refers not only to the force required to tighten the screw but also to the strain of trying to turn the screw when it is stuck resisting the tightening force exerted thereon. In a further example, where the worker handles a workpiece material as the work target, work intensity refers not only to the force required to cut the workpiece material but also to the force of trying to cut the workpiece material when it is not being cut resisting the cutting force exerted thereon. In this manner, work intensity is a concept that includes the orders of magnitude of various forces acting on the work target by the worker.

The action recognition model 114 may be a database for example, which stores the model by which the action recognition section 112 determines and recognizes the action of the worker.

The work intensity determination model 115 may be a database for example, which stores the model by which the work intensity determination section 113 determines the work intensity of the worker.

The storage section 116 may be a RAM (Random Access Memory) for example, which provides a work area in which the CPU executes programs.

The control section 117 may be a CPU for example, which executes the programs of the computer 110.

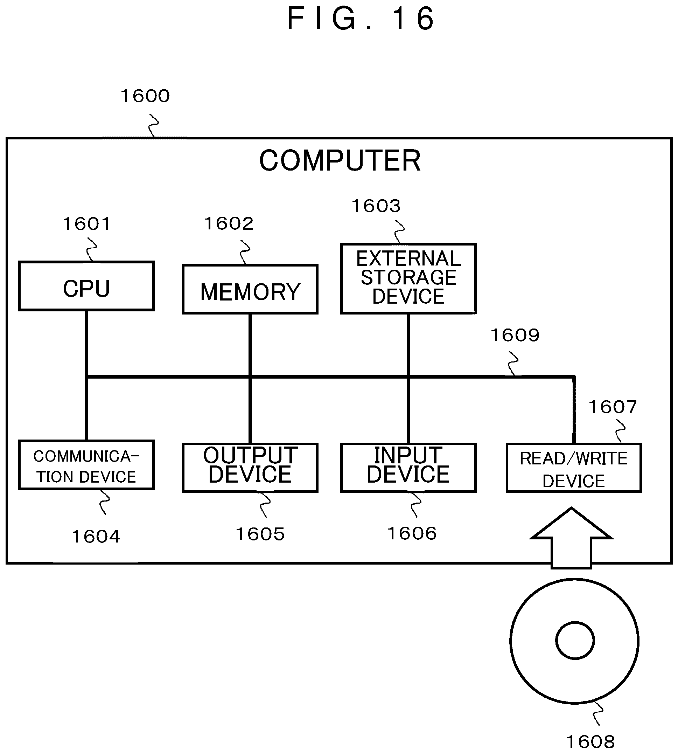

The computer 110 in FIG. 1 may be implemented by a common computer 1600 that includes, as depicted in FIG. 16 (computer schematic diagram), a CPU 1601, a memory 1602, an external storage device 1603 such as a HDD (Hard Disk Drive), a read/write device 1607 that writes and reads information to and from portable storage media 1608 such as a CD (Compact Disk) or a USB memory, an input device 1606 such as a keyboard and a mouse, an output device 1605 such as a display unit, a communication device 1604 such as a NIC (Network Interface Card) for connecting to a communication network, and an internal communication line (called a system bus) 1609 such as the system bus for interconnecting these components.

Various data stored into each system or device, or used in processing may be implemented by the CPU 1601 reading for utilization from the memory 1602 or external storage device 1603. Each functional section (to be discussed later) included in each system or device may be implemented by the CPU 1601 loading predetermined programs from the external storage device 1603 into the memory 1602 and executing the loaded programs.

The above-mentioned predetermined programs may alternatively be stored (downloaded) into the external storage device 1603 from the storage media 1608 via the read/write device 1607 or from the network via the communication device 1604, before being loaded into the memory 1602 and executed by the CPU 1601. As another alternative, the programs may be directly loaded into the memory 1602 from the storage media 1608 via the read/write device 1607 or from the network via the communication device 1604, before being executed by the CPU 1601.

Whereas what is described below is an example in which the work action recognition system 100 is configured with a single computer, part or all of the functions of the action recognition section 112 and work intensity determination section 113 may alternatively be distributed in one or multiple computers as in a cloud setup and made to communicate with each other over the network to provide similar functions. Whereas the information presentation device 101 is indicated as a device different from the computer 110 in FIG. 1, the information presentation device 101 may be integrated with the computer 110 instead.

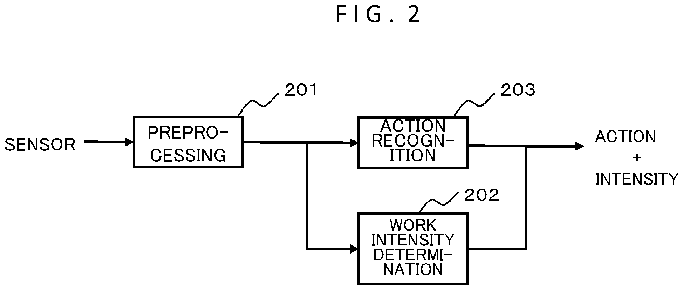

FIG. 2 is a diagram depicting a typical processing procedure performed by the work action recognition system 1000 according to the first embodiment. In the work action recognition system 1000, as depicted in FIG. 2, the control section 117 first performs preprocessing on sensor values constituting the information detected by the sensor (e.g., acceleration at given points in time) (step 201). The preprocessing involves carrying out processes intended to improve the accuracy of sensor values input to the system, such as normalization of the sensor values, calculation of an average value per unit time, and elimination of faulty values that fail to meet predetermined criteria.

The work intensity determination section 113 reads the preprocessed sensor values and the work intensity determination model 115 and, using a training method such as Deep Learning, determines the work intensity of the worker and outputs the result of the determination (step 202) .

The action recognition section 112 reads the preprocessed sensor values and the action recognition model 114 and, using a training method such as Rula (Rapid upper limb assessment) or Owas (Ovako working posture analyzing system), determines and recognizes the action of the worker and outputs the result of the determination and recognition (step 203).

In the present embodiment, the processes of steps 201 through 203 are carried out. The control section 117 associates the result of the determination by the work intensity determination section 113 with the result of the recognition by the action recognition section 112, and outputs the resulting association to the information presentation device 101. This makes it possible to grasp with what intensity and in what action the worker is working on the work target. For example, in the case where the worker walks while carrying a package, it is possible to grasp whether the steps of the walk are "light" or "heavy."

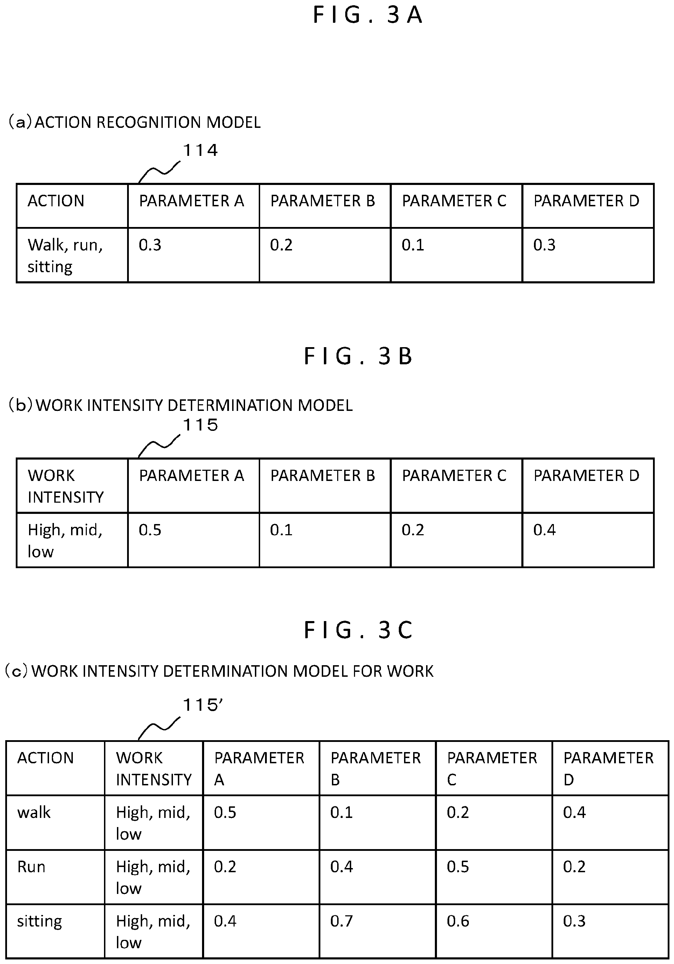

FIGS. 3A through 3C are tabular views indicating typical models used by this system. (a) in FIG. 3A indicates a typical action recognition model 114, and (b) in FIG. 3B indicates a typical work intensity determination model 115.

As indicated in (a) in FIG. 3A, the action recognition model 114 stores the actions of the worker such as "Walk," "Run," and "Sitting," along with parameters (e.g., weight values and coefficients) for use by the above-mentioned training method.

As indicated in (b) in FIG. 3B, the work intensity determination model 115 stores levels of work intensity such as "High," "Middle," and "Low," along with parameters (e.g., weight values and coefficients) for use by the above-mentioned training method.

It is explained above that, in the present embodiment, the actions or the work intensity of the worker are associated with parameters. Alternatively, as indicated in (c) in FIG. 3C, the work intensity may be determined for each type of action of the worker. As listed in (c) in FIG. 3C, three levels of work intensity such as "High," "Middle," and "Low" may be defined for the type of action "Walk." The above-mentioned parameters may then be stored in the form of the work intensity determination model 115' according to the work intensity.

As described above, the work action recognition system 1000 according to the present embodiment includes the action recognition section 112 that recognizes the action of the worker on the basis of the information detected by the sensor 100 worn by the worker and of a predetermined action recognition model (e.g., action recognition model 114); the work intensity determination section 113 that determines the intensity of the work performed by the worker on the basis of the information detected by the sensor 100 and of a predetermined work intensity determination model (e.g., work intensity determination model 115); and the control section 117 that outputs the associations made between the action of the worker obtained by the action recognition section 112 and the intensity of the work carried out by the worker and acquired by the work intensity determination section 113. This makes it possible to grasp with what work intensity and in what action the worker is working.

### Second embodiment

It is explained above that the first embodiment outputs to the information presentation device the associations made between the action and the work intensity of the worker using a predetermined action recognition model and a predetermined work intensity determination model. Alternatively, instead of using the predetermined action recognition model and work intensity determination model, trained models may be used to improve the accuracy of determination. Explained below is a case in which the models are trained.

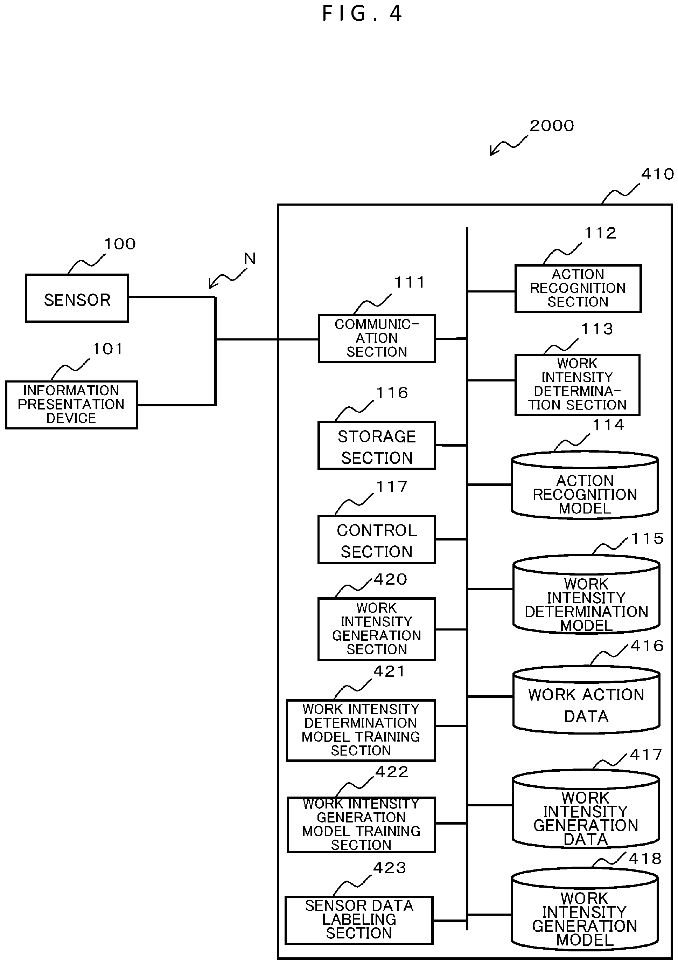

FIG. 4 is a diagram depicting a typical configuration of a work action recognition system 2000 according to a second embodiment of the present invention. As depicted in FIG. 4, a computer 410 of the work action recognition system 2000 includes, in addition to the functional sections discussed in connection with the first embodiment, a work intensity generation section 420, a work intensity determination model training section 421, a work intensity generation model training section 422, and a sensor data labeling section 423. The computer 410 further stores work action data 416, work intensity generation data 417, and a work intensity generation model 418.

The work intensity generation section 420 may be a program executed by the CPU, for example. The work intensity generation section 420 performs a work intensity data generation process to generate the work intensity generation data 417 using the work action data 416 and the work intensity generation model 418.

The work intensity determination model training section 421 may be a program executed by the CPU for example. The work intensity determination model training section 421 performs a work intensity determination model training process to generate the work intensity determination model 115 from the work intensity generation data 417.

The work intensity generation model training section 422 may be a program executed by the CPU, for example. The work intensity generation model training section 422 performs a work intensity determination model training process to generate the work intensity generation model 418 from the work action data 416.

The sensor data labeling section 423 may be a program executed by the CPU, for example. The sensor data labeling section 423 performs a labeling process to provide labels indicative of the type of action and the work intensity of the worker with respect to the information detected by the sensor 100, on the basis of the operations carried out by the worker. For example, the worker may take a video of his or her work with a camera, not depicted, by use of the computer 410, and by operating the information presentation device 101 while verifying the captured video and the sensor values constituting the information detected by the sensor 100, the worker performs labeling to associate the sensor values with the type of action and the work intensity of the worker.

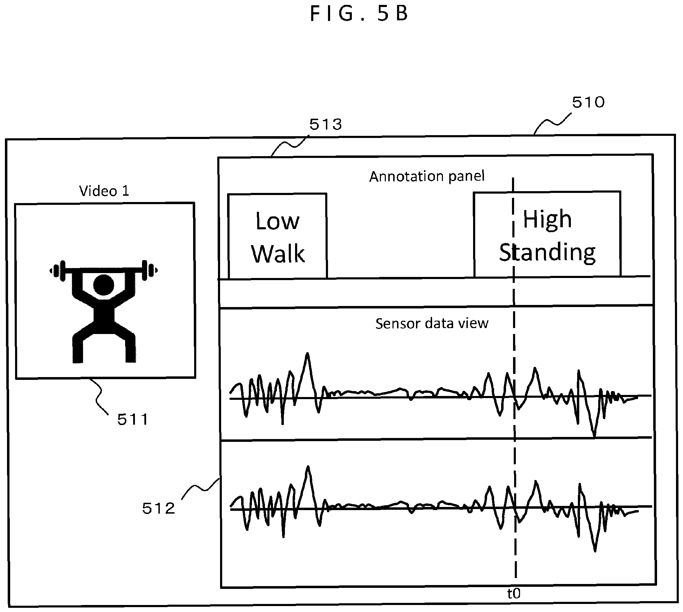

FIG. 5B is a diagram depicting a typical screen (annotation panel) displayed on the information presentation device 101 by the sensor data labeling section 423 for purpose of labeling. As depicted in FIG. 5B, the annotation panel 510 includes a work display region 511 for displaying the video capturing the work performed by the worker, a sensor value display region 512 for displaying transitions of the sensor values detected by the sensor 100 during work, and a labeling information input region 513 for inputting, in an association manner, the type of action and the work intensity of the worker working while verifying the work display region 511 and the sensor value display region 512.

In FIG. 5B, the work display region 511 displays the video capturing the worker during work (Video 1), and the sensor value display region 512 displays the sensor values obtained by two sensors 100 worn by the worker, the sensor values being displayed on the screen from right to left in chronological order. It is indicated that "Standing" is input as the type of action of the worker at a point in time t0 and that "High" is input as the work intensity of the worker at the point in time t0. In the sensor value display region 512, the type of action "Walk" and the work intensity "Low" of the worker are the information at the start of work. In this manner, the worker labels the sensor values with the type of action and the work intensity of the worker at various points in time while verifying the work display region 511 and the sensor value display region 512.

As described above, the present embodiment includes the input section (e.g., touch panel of the information presentation device 101) to which the worker inputs labeling information in which the type of action performed by the worker is associated with the work intensity of the worker (e.g., above described information in which type of action and the work intensity of the worker are associated with each other), and the work intensity determination model training section 421 generates the work action data 416 by associating the information detected by the sensors 100 with the labeling information input from the input section. This makes it possible to obtain the data in which the sensor values are associated with the type of action and the work intensity of the worker, based on the worker's own perceptions. The data may be used as the work action data 416, as will be explained later.

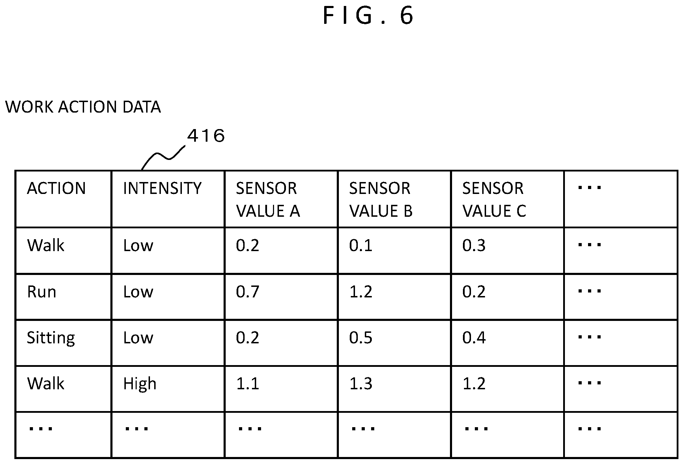

The work action data 416 may constitute a database for example, which stores the data in which the sensor values are associated with the actions of the worker at given points in time. Specific examples of the work action data 416 will be discussed later with reference to FIG. 6.

The work intensity generation data 417 may constitute a database for example which, as will be discussed later, stores the data in which the work intensity generation model 418 having learned from the work action data 416 is reflected on the work action data 416. The layout of the work intensity generation data 417 is similar to that of the work action data 416.

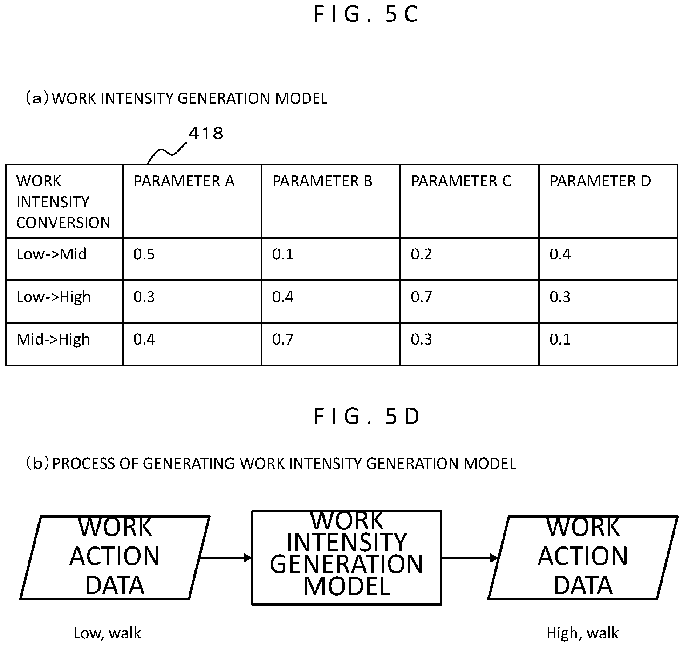

The work intensity generation model 418 may constitute a database for example, which stores the data in which work intensity conversions indicative of how the work intensity varies are associated with the parameters (e.g., weight values and coefficients) to be used by the above-mentioned training method. A specific example of the work intensity generation model 418 will be discussed later with reference to FIG. 5C.

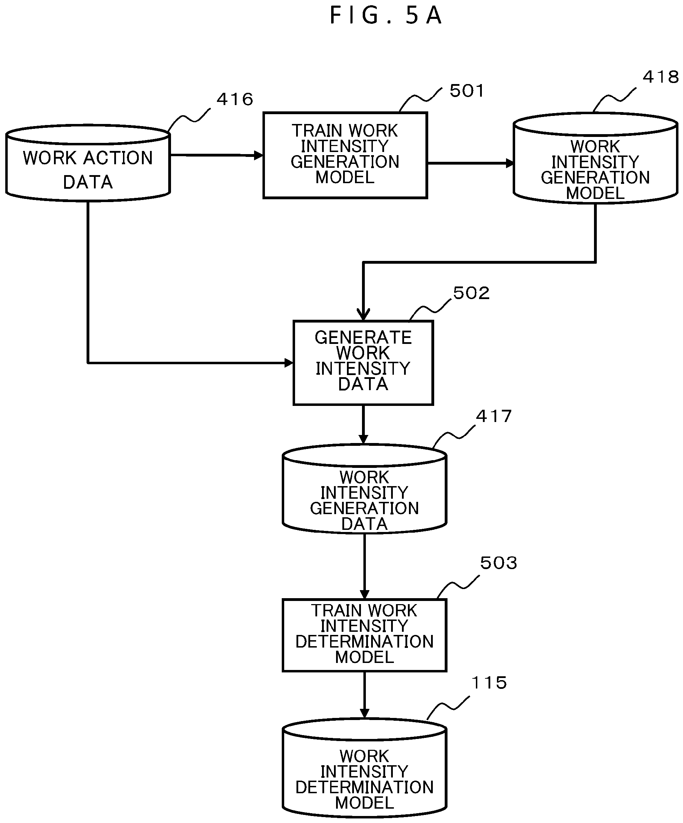

FIG. 5A is a diagram depicting a typical processing procedure performed by the work action recognition system 2000 according to the second embodiment. In the work action recognition system 2000, as depicted in FIG. 5A, the work intensity generation model training section 422 first reads the work action data 416 to generate the work intensity generation model 418 (step 501).

FIG. 6 is a tabular view listing typical work action data 416. The work action data 416 provides associations made between the levels of work intensity and the sensor values corresponding to the levels, with respect to diverse types of action performed by each worker. In the work action data 416, as listed in FIG. 6, the action, work intensity, and multiple sensor values of the worker are stored in association with each other. For example, FIG. 6 indicates that senor values A, B, and C in effect when working in the action "Walk" with the work intensity "Low" are "0.2," "0.1," and "0.3," respectively. As indicated in FIG. 5B, the work action data 416 is generated by the sensor data labeling section 423.

FIG. 5C is a tabular view indicating a typical work intensity generation model 418 and a typical process for generating the model. The work intensity generation model 418 is a model that learns the transition of work intensity of the worker. As listed in (a) in FIG. 5C, the work intensity generation model 418 has the types of work intensity transitions stored in association with the parameters used by the above-mentioned training method (e.g., weight values and coefficients). For example, it is indicated that parameters A, B, C and D for estimating a change of the work intensity of the worker from "Low" to "Middle" are "0.5," "0.1," "0.2," and "0.4," respectively.

The values of these parameters are estimated anew by use of the above-mentioned training method every time the work action data 416 is input to the work intensity generation model 418, as depicted in (b) in FIG. 5D. (b) in FIG. 5D indicates that a newly generated work intensity generation model 418 is trained so as to replace the work action data 416 about the worker with "Low" work intensity in "Walk" action with the work action data 416 on the worker with "High" work intensity in "Walk" action.

Returning to FIG. 5A, the work intensity generation model training section 422 generates the work intensity generation model 418 in step 501. In turn, the work intensity generation section 420 generates the work intensity generation data 417 using the work action data 416 and the trained work intensity generation model 418 (step 502). The work intensity generation data 417 has the items similar to those of the work action data 416. The trained work intensity generation model 418 has converted the "action" and "work intensity" items in the work action data 416 to those learned anew.

The work intensity determination model training section 421 proceeds to generate the work intensity determination model 115 from the work intensity generation data 417 generated in step 502 (step 503). In the work intensity determination model 115, as explained above in connection with the first embodiment, the values of the parameters corresponding to the work intensity become those learned anew, in the present embodiment.

As described above, the work action recognition system 2000 of the present embodiment includes: the work intensity generation model training section 422 which, given the work action data 416 in which the action of the worker is associated with the information detected by the sensors 100, generates the work intensity generation model 418 in which manners of change in the work intensity are associated with the parameters used by the predetermined training method; the work intensity generation section 420 that generates the work intensity generation data 417 in which the trained work intensity generation model 418 is reflected on the work action data 416, based on the work action data 416 and on the work intensity generation model 418 trained by the work intensity generation model training section 422; and the work intensity determination model training section 421 that generates, as the predetermined work intensity determination model 115, the work intensity generation data 417 generated by the work intensity generation section 420. This makes it possible to accurately grasp with what work intensity the worker is acting, compared with the first embodiment.

### Third embodiment

In the first and the second embodiments, the action recognition model and the work intensity determination model are used to output to the information presentation device the associations made between the action and the work intensity of the worker. Preferably, however, the information output to the information presentation device may be such as to include scoring of the work load on the worker in a manner allowing the resulting scores to be visually grasped. Explained below is thus a case where information regarding the load on the worker is output to the information presentation device.

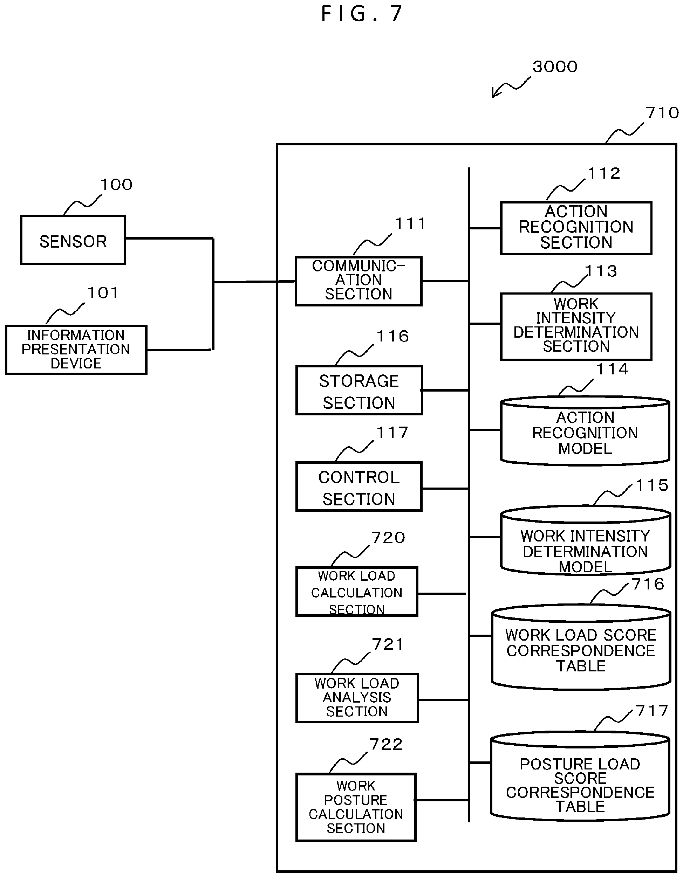

FIG. 7 is a diagram depicting a typical configuration of a work action recognition system 3000 according to a third embodiment of the present invention. As depicted in FIG. 7, a computer 710 of the work action recognition system 3000 includes, in addition to the functional sections discussed in connection with the first embodiment, a work load calculation section 720, a work load analysis section 721, and a work posture calculation section 722. Further, the computer 710 stores a work load store correspondence table 716 and a posture load score correspondence table 717.

The work load calculation section 720 may be a program executed by the CPU, for example. The work load calculation section 720 performs a work load calculation process to calculate work load values scoring the work load on the worker, by use of the work intensity of the worker determined by the work load determination section 113 using a training method such as Deep Learning, the action of the worker recognized by the action recognition section 112 using a training method such as Rula or Owas, and the work load score correspondence table 716.

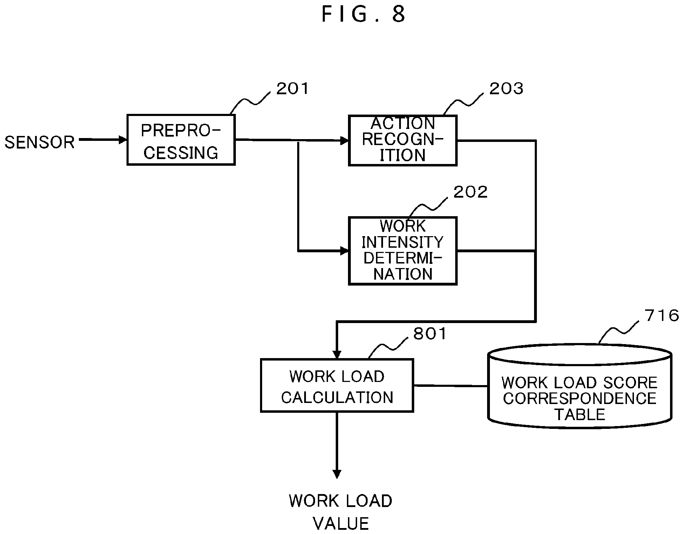

FIG. 8 is a diagram depicting a typical processing procedure performed by the work action recognition system 3000 according to the third embodiment. The processes "preprocessing," "action recognition," and "work intensity determination" in FIG. 8 are similar to those of the first embodiment and thus will not be discussed further. As depicted in FIG. 8, when the processes "action recognition" and "work intensity determination" are carried out in the work action recognition system 3000, the work load calculation section 720 reads the load scores corresponding to the results of these processes from the work load score correspondence table 716 for calculation as the work load values of the worker (step 801). The load scores are values that score the load on the worker.

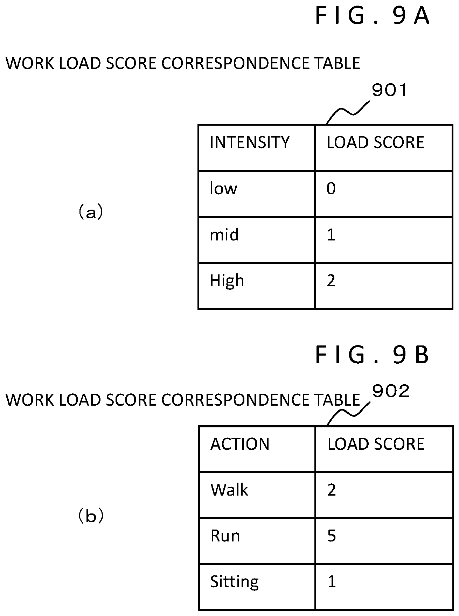

FIGS. 9A and 9B are tabular views indicating examples of the work load score correspondence table 716. The work load score correspondence table 716 constitutes data defining the load score for work intensity and each action of the worker. (a) in FIG. 9A lists typical data in which the work intensity of the worker is associated with the corresponding load scores (intensity load score correspondence table), and (b) in FIG. 9B lists typical data in which the actions of the worker are associated with the corresponding load scores (action load score correspondence table).

In the intensity load score correspondence table 901, the work intensity of the worker and the correspondence load scores are stored in association with each other, as indicated in (a) in FIG. 9A. For example, in the case where the work intensity of the worker is "Low" in (a) in FIG. 9A, the corresponding load score is indicated to be "0." In the action load score correspondence table 902, the actions of the worker and the corresponding load scores are stored in association with each other, as indicated in (b) in FIG. 9B. For example, in the case where the action of the worker is "Walk" in (b) in FIG. 9B, the corresponding load score is indicated to be "2."

In this manner, the present embodiment defines the load score for each action and work intensity of the worker, thereby allowing the work load on the worker to be determined in accordance with the load scores. Furthermore, in addition to the work load score correspondence table 716, the present embodiment uses the posture load score correspondence table 717 to calculate the load values of the worker in consideration of the worker's postures.

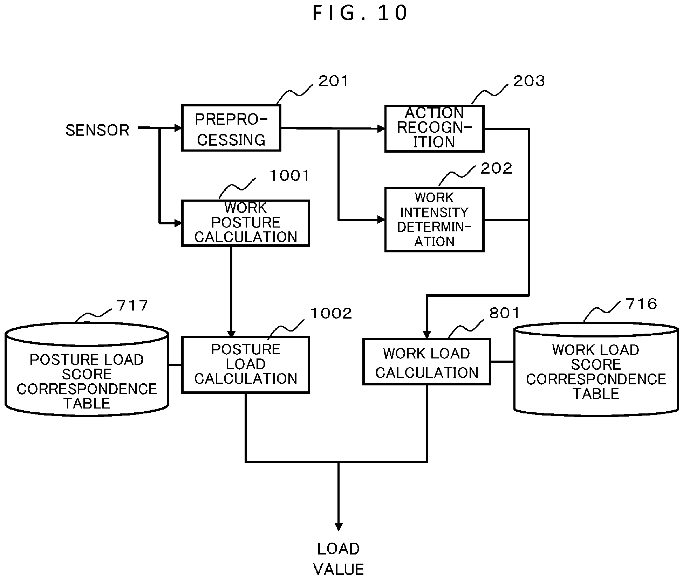

FIG. 10 is a diagram depicting another typical processing procedure performed by the work action recognition system 3000 as the third embodiment. The processes "preprocessing," "action recognition," and "work intensity determination" in FIG. 10 are similar to those of the first embodiment and the process "work load calculation" is similar to that in FIG. 8, so that these processes will not be discussed further. In the work action recognition system 3000, as depicted in FIG. 10, the control section 117 performs "preprocessing" and the work posture calculation section 722 calculates the work posture of the worker using the sensor values constituted by the information detected by the sensor 100 (e.g., acceleration at given points in time) (step 1001). The work posture may be calculated by use of various existing known techniques including one that extracts information regarding the skeleton of the worker for calculation purposes.

The work posture calculation section 722 calculates the load of the work posture of the worker by referencing the posture of the worker obtained by calculation in step 1001 as well as the posture load score correspondence table 717 (step 1002). The work load calculation section 720 adds up the load score of the work posture calculated by the work posture calculation section 722 and the load score of the work intensity and action of the worker calculated in step 801, thereby providing the final load score of the worker.

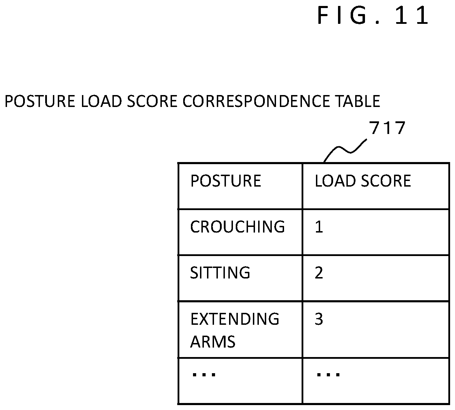

FIG. 11 is a tabular view indicating a typical posture load score correspondence table 717. The posture load score correspondence table 717 lists data defining the load scores with regard to the work postures of the worker. Indicated in FIG. 11 are typical data in which the work postures of the worker are associated with load scores (i.e., work posture load score correspondence table). In the posture load score correspondence table 717, as depicted in FIG. 11, the postures of the worker and the load scores corresponding to such postures are stored in association with each other. For example, in FIG. 11, when the worker is in the posture "crouching," the corresponding load score is indicated to be "1."

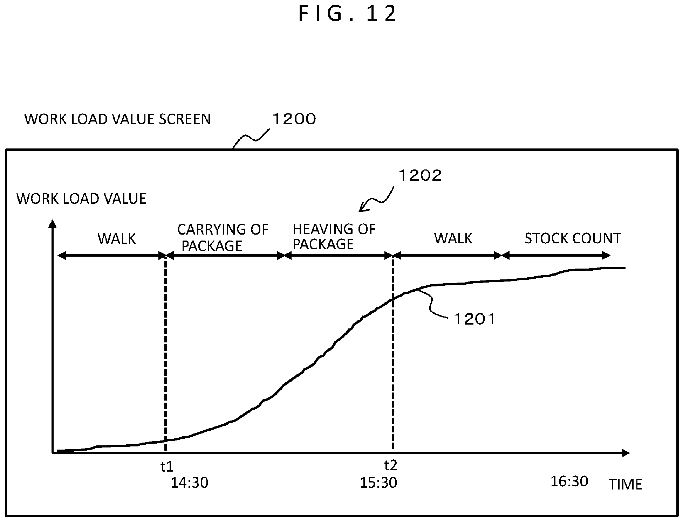

FIG. 12 is a diagram depicting a typical screen (work load value screen) which indicates a transition of the load on the worker (work load values) and which is output to the information presentation device by the work load calculation section 720. As depicted in FIG. 12, with the horizontal axis denoting time and with the vertical axis representing work load values, the work load value screen indicates the transition of the work load values using a graph 1201. Actions 1202 of the worker are displayed in association with the graph 1201. In FIG. 12, for example, the actions of the worker in a time slot from time t1 to time t2 are "carrying of package" and "heaving of package." It is indicated that the work load values are on the increase in transition from "carrying of package" to "heaving of package." In this manner, the work action recognition system 3000 of the present embodiment includes the work load calculation section 720 that calculates the work load on the worker on the basis of the result of the recognition by the action recognition section 112, the result of the determination by the work intensity determination section 113, and the work load score correspondence table 716 in which the load scores scoring the load on the worker are associated with each action of the worker representing the above result of the recognition and with work intensity of the worker representing the above result of the determination. Further, the work load calculation section 720 outputs, to the information presentation device 101, a work load value screen 1200 indicating a chronological transition of the work load on the worker. Thus, when displayed on the screen, the transition of the work load values of the worker can be grasped at a glance. Furthermore, the display on the screen of the associations between the work load values and the actions of the worker makes it possible easily to grasp the trend of work load values such as one indicating a specific action of the worker being associated with high (or low) work load values.

Also included is the work posture calculation section 722 that calculates the load on the worker in the work postures on the basis of the posture load score correspondence table 717 making the associations between the work postures of the worker calculated using the information detected by the sensor 100 on one hand, and the load scores scoring the load on the worker in the calculated work postures of the worker on the other hand. The work load calculation section 720 may further calculate the work load on the worker using the load on the worker in the work postures, and output the work load value screen 1200 to the information presentation device 101. This makes it possible to calculate the work load in consideration of the work postures of the worker, and provide an at-a-glance picture of the transition of the work load values of the worker as well as the work postures of the worker. It is thus easy to grasp the trend of the work load values involved.

Indicated in FIG. 12 is the display of the transition of the final load on the worker (work load values) by use of the values adding up those of the scored actions, work intensity, and work postures of the worker. In some cases, however, it may be desired to grasp the specifics on which the work load values are based. In such a case, the work load calculation section 720 may cause the information presentation device to display, on the same screen as (or different screen from) the work load value screen indicated in FIG. 12, the work load store correspondence table 716 in FIGS. 9A and 9B and the posture load score correspondence table 717 in FIG. 11. This makes it possible easily to grasp the breakdown of the final work load values, such as percentages of the load with respect to the actions, work intensity, and work postures of the worker.

### Fourth embodiment

The system of the first through the third embodiments is indicated to be configured such that the work intensity of a given worker can be estimated.
However, this system is also capable of managing the work load of a worker as well as his or her action and work with respect to each of multiple workers. Explained below is thus the case where this system is applied to multiple workers.

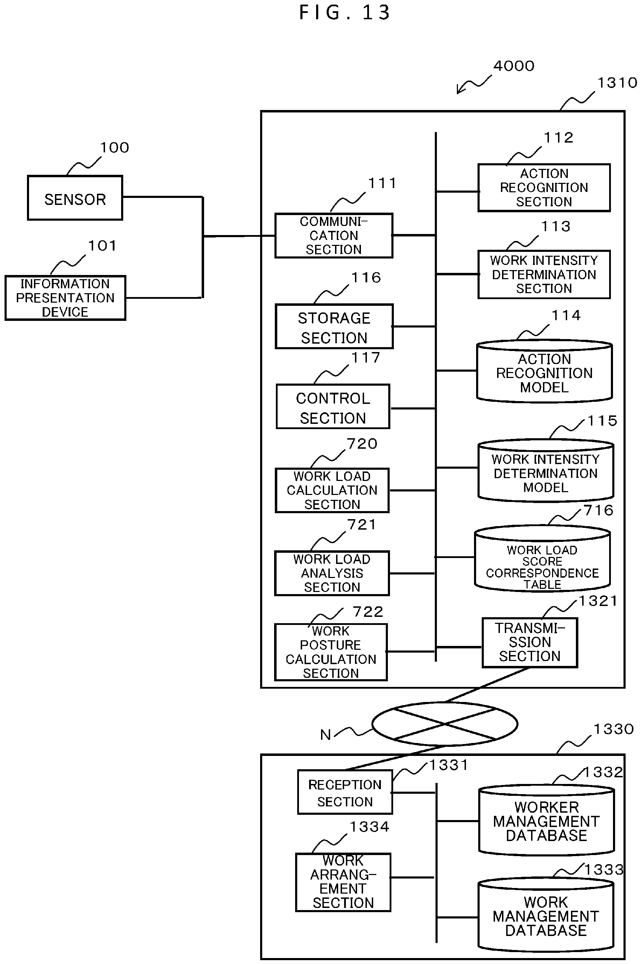

FIG. 13 is a diagram depicting a typical configuration of a work action recognition system 4000 as a fourth embodiment of the present invention. As depicted in FIG. 13, a computer 1310 of the work action recognition system 4000 has a transmission section 1321 in addition to the functional sections discussed in connection with the third embodiment. Whereas FIG. 13 indicates the sensor 100, information presentation device 101, and computer 1310 in a single set for use with a given worker, the present embodiment is assumed to have this configuration for each of multiple workers. The computers for the respective workers are assumed to send and receive diverse information to and from each other via a network N.

For example, the transmission section 1321 may be a NIC transmitting diverse information to a management server 1330. In the present embodiment, the functional section for conducting communication between the computer 1310 and the management server 1330 is described as the transmission section. Alternatively, there may be provided a communication section including the function for receiving various information from the management server 1330.

The work action recognition system 4000 further includes the management server 1330 connected to the computer 1310 via the network N. In terms of hardware, the management server 1330 may be a computer having a configuration similar to that of the computer 1310.

As depicted in FIG. 13, the management server 1330 includes a reception section 1331, a worker management database 1332, a work management database 1333, and a work arrangement section 1334.

The reception section 1331 may be a NIC for example, which receives diverse information transmitted from the computer 1310. In the present embodiment, the functional section for conducting communication between the computer 1310 and the management server 1330 is described as the transmission section. Alternatively, there may be provided a communication section including the function for transmitting various information to the computer 1310.

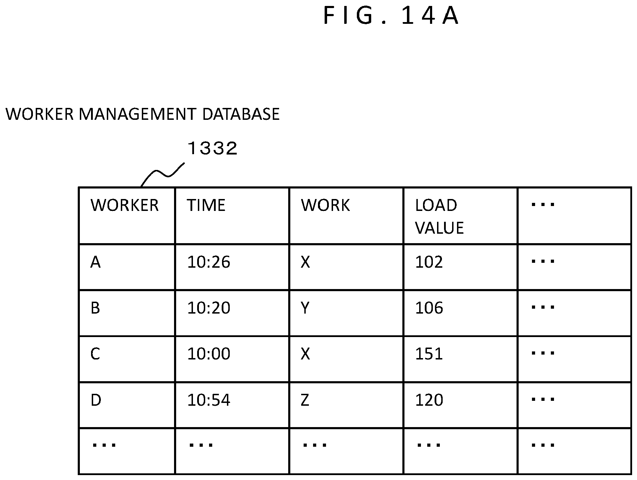

The worker management database 1332 stores the work of each of multiple workers as well as the load value of such work in a time-related manner. A specific example of the worker management database 1332 will be discussed later with reference to FIG. 14A. FIG. 14A indicates an example in which the target to be managed is "work." Since work is constituted by one or multiple actions, "actions" corresponding to "work" may also be stored.

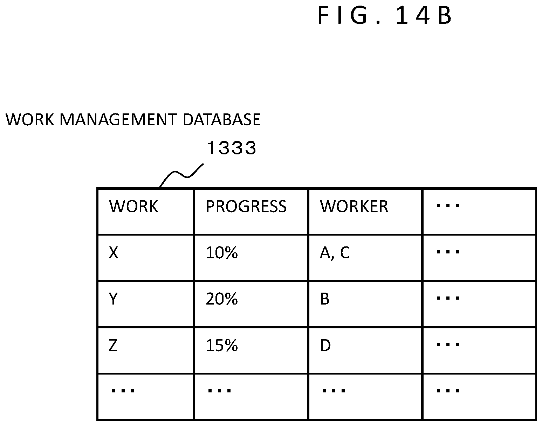

The work management database 1333 stores the types of work, the progress of each work, and the worker performing each work in a mutually associated manner. A specific example of the work management database 1333 will be discussed later with reference to FIG. 14B. FIG. 14B indicates an example in which the target to be managed is "work." Alternatively, the target to be managed may be "action."

FIG. 14A is a tabular view giving an example of the worker management database 1332. The worker management database 1332 constitutes data storing the load values of the work at given points in time with regard to each worker. FIG. 14A indicates, for example, that worker A performs work "X" at 10:26 with a load value of "102." The work "X" may include, for example, one or multiple actions such as "carrying of package" and "heaving of package." In FIG. 14A, the types of work and the actions are associated with each other beforehand. As discussed in connection with the third embodiment, the load value adding up the load values of different actions is calculated as the load value of the work.

FIG. 14B is a tabular view giving an example of the work management database 1333. The work management database 1333 constitutes data storing the progress of the work performed by workers. FIG. 14B indicates, for example, that work X is performed by workers A and C and that the current progress status is 10%. As in the case of FIG. 14A, the work "X" includes, for example, one or multiple actions such as "carrying of package" and "heaving of package." In FIG. 14B, the types of work, the actions, and the workers are associated with each other beforehand. For example, it supposed a case where that work performed by workers A and C is made up of such actions that "walk," "carrying of package," "heaving of package," "walk," and "stock count" is defined beforehand. If the work is completed up to the actions "walk" and "carrying of package," for example, the progress is calculated to be 400. With regard to whether each of the actions making up the work is completed or not, one type of action may be determined to be completed when the type of action of the worker input from the labeling information input region 513 on the annotation panel 510 is changed from the one type to another type, as indicated in FIG. 5B for example.

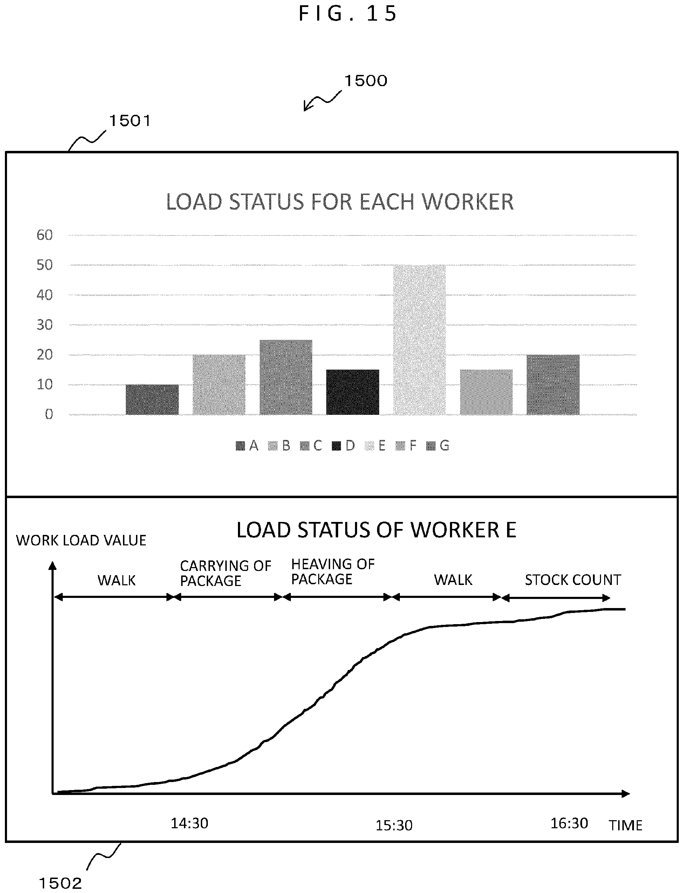

FIG. 15 is a diagram depicting a typical screen (load management screen) 1500 that is output on a display device (or information presentation device), not depicted, by the work arrangement section 1334 indicating the data stored in the worker management database 1332 in FIG. 14A as well as the data stored in the work management database 1333 in FIG. 14B. As depicted in FIG. 15, the load management screen 1500 includes a load status display region 1501 and a load status transition display region 1502, the load status display region 1501 being displayed per worker on the basis of the data stored in the worker management database 1332, the load status transition display region 1502 indicating the transition of work load values of a given worker and being displayed on the basis of the data stored in the work management database 1333.

For example, in FIG. 15, the load status of each of workers A through G at a given point in time is displayed in the load status display region 1501. Further, for example, with regard to the load status of the worker E, through selection or the like of a bar in the bar chart of the load status display region 1501, a transition of load values during a given time slot in reference to a selected point in time (e.g., over one hour before and after the selected point in time) is displayed in the load status transition display region 1502. The selection may be carried out, for example, by an operator touching a touch panel or like interface that may be attached to the display device displaying the load management screen 1500.

As described above, the work action recognition system 4000 of the present embodiment includes the computer 1310 that includes, for each of multiple workers, the action recognition section 112, work intensity determination section 113, control section 117, and work load calculation section 720; and the management server 1330 that includes the storage section (e.g., storage device storing the worker management database 1332) storing, for each worker, the work load on the worker received from each computer 1310 via the network N, and the work arrangement section 1334 retrieving the work load per worker from the storage section and outputting to the display device (e.g., display unit connected to the management server 1330) the load management screen 1500 including the load status display region 1501 indicating the retrieved work load per worker. This configuration provides an at-a-glance picture of the work status regarding each worker.

Also, the work arrangement section 1334 of the management server 1330 outputs to the above-described display device the load management screen 1500 including the load status transition display region 1502 indicating the chronological transition of the work load on the worker selected in the load status display region 1501. This makes it possible to easily grasp the transition of the load status regarding the selected worker.

### Reference Signs List

1000, 2000, 3000, 4000: Work action recognition system
100: Sensor 100
101: Information presentation device
110: Computer
111: Communication section
112: Action recognition section
113: Work intensity determination section
114: Action recognition model
115: Work intensity determination model
116: Storage section
117: Control section
416: Work action data
417: Work intensity generation data
418: Work intensity generation model
420: Work intensity generation section
421: Work intensity determination model training section
422: Work intensity generation model training section
423: Sensor data labeling section
716: Work load score correspondence table
720: Work load calculation section
721: Work load analysis section
722: Work posture calculation section
717: Posture load score correspondence table
1321: Transmission section
1330: Management server
1331: Reception section
1332: Worker management database
1333: Work management database
1334: Work arrangement section
N: Network

## Claims

1. A work action recognition system comprising:
an action recognition section that recognizes an action of a worker on a basis of information detected by a sensor worn by the worker and a predetermined action recognition model;
a work intensity determination section that determines work intensity of work performed by the worker, on a basis of the information detected by the sensor and a predetermined work intensity determination model; and
a control section that associates the action of the worker obtained by the action recognition section with the work intensity of the work performed by the worker, the work intensity being obtained by the work intensity determination section, and outputs the association.

2. The work action recognition system according to claim 1, comprising:
a work intensity generation model training section that generates a work intensity generation model in which a manner of change in the work intensity is associated with a parameter used by a predetermined training method, the work intensity generation model being generated from work action data in which the action of the worker is associated with the information detected by the sensor;
a work intensity generation section that, on a basis of the work action data and the work intensity generation model trained by the work intensity generation model training section, generates work intensity generation data in which the trained work intensity generation model is reflected on the work action data; and
a work intensity determination model training section that generates, as the predetermined work intensity determination model, the work intensity generation data generated by the work intensity generation section.

3. The work action recognition system according to claim 2, comprising:
an input section that inputs labeling information in which a type of action of the worker is associated with the work intensity, wherein
the work intensity determination model training section generates the work action data by associating the information detected by the sensor with the labeling information input from the input section.

4. The work action recognition system according to claim 1, comprising:
a work load calculation section that calculates work load on the worker on a basis of a result of recognition by the action recognition section, a result of determination by the work intensity determination section, and a work load score correspondence table in which a load score for scoring the load on the worker is associated with the action of the worker representing the result of the recognition and with the work intensity of the worker representing the result of the determination.

5. The work action recognition system according to claim 4, comprising:
a work posture calculation section that calculates a work posture of the worker using the information detected by the sensor, and on a basis of a posture load score correspondence table in which the load score for scoring the load on the worker is associated with the calculated work posture of the worker, calculates the load on the worker in the work posture, wherein
the work load calculation section further calculates the work load on the worker using the load on the worker in the work posture.

6. The work action recognition system according to claim 4, wherein
the work load calculation section outputs to an information presentation device a work load value screen indicating a chronological transition of the work load on the worker.

7. The work action recognition system according to claim 4, comprising:
a computer including, for each of a plurality of the workers, the action recognition section, the work intensity determination section, the control section, and the work load calculation section; and
a management server including a storage section that stores, for each of the workers, the work load on the worker received from each of the computers via a network, and a work arrangement section that retrieves the work load on each worker, the work load being stored in the storage section, and outputs to a display device a load management screen including a load status display region indicating the retrieved work load per worker.

8. The work action recognition system according to claim 7, wherein
the work arrangement section of the management server outputs to the display device the load management screen including a load status transition display region indicating a chronological transition of the work load on the worker selected in the load status display region.

9. A work action recognition method performed by use of a computer, the work action recognition method comprising:
by an action recognition section, recognizing an action of a worker on a basis of information detected by a sensor worn by the worker and a predetermined action recognition model;
by a work intensity determination section, determining work intensity of work performed by the worker, on a basis of the information detected by the sensor and a predetermined work intensity determination model; and
by a control section, associating the action of the worker obtained by the action recognition section with the work intensity of the work performed by the worker, the work intensity being obtained by the work intensity determination section, and outputting the association.
